(19) Europäisches Patentamt
European Patent Office
Office européen des brevets

(11) Publication number: **0 448 748 A1**

# (12) EUROPEAN PATENT APPLICATION

(21) Application number: **90105756.2**

(51) Int. Cl.5: **C07C 315/00**

(22) Date of filing: **27.03.90**

(43) Date of publication of application:
**02.10.91 Bulletin 91/40**

(84) Designated Contracting States:
**BE DE FR GB IT**

(71) Applicant: **SANKO CHEMICAL COMPANY LTD.**
**8-16, Tooricho,**
**Kurume-shi, Fukuoka(JP)**

Applicant: **NEW JAPAN CHEMICAL CO.,LTD.**
**13, Yoshijima Yaguracho Fushimi-ku**
**Kyoto-shi Kyoto 612(JP)**

(72) Inventor: **Saito, Toranosuke, c/o Sanko Chemical Co.-Ltd.**
**Osaka Kenkyusho nai, 10-24, Itsukaichi 1-chome**
**Ibaraki-shi, Osaka(JP)**

(74) Representative: **Gervasi, Gemma et al**
**NOTARBARTOLO & GERVASI Srl Viale Bianca Maria 33**
**I-20122 Milan(IT)**

## (54) Method for preparing aromatic sulfones.

(57) Herein disclosed are a method for preparing an aromatic sulfone comprising the step of condensing, by heating, an aromatic compound with sulfuric acid or an aromatic sulfonic acid in the presence of a heteropoly-acid and orthophosphoric acid; and a method for preparing an aromatic sulfone comprising the steps of: condensing, by heating, an aromatic compound with sulfuric acid or an aromatic sulfonic acid in the presence of a heteropoly-acid and orthophosphoric acid; adding water and optionally an aromatic compound to the resulting reaction mixture to separate it into (i) a mixture of the aromatic sulfone and unreacted aromatic compound and (ii) an aqueous solution containing the heteropoly-acid, orthophosphoric acid and aromatic sulfonic acid, to thus recover the same. The aqueous solution containing the heteropoly-acid, orthophosphoric acid and aromatic sulfonic acid can be recyled or reused in the subsequent condensation reaction.

EP 0 448 748 A1

## BACKGROUND OF THE INVENTION

The present invention relates to a method for preparing aromatic sulfones. The aromatic sulfones have a wide variety of industrial applications as high boiling point solvents, plasticizers, dye-assistants as well as intermediates for medicines, dyes or synthetic resins.

There have been known a variety of methods for preparing aromatic sulfones such as those listed below:

1. Those based on the oxidation of an aromatic sulfide or an aromatic sulfoxide;
2. Those based on the condensation of an aromatic compound with an aromatic sulfonyl chloride in the presence of a Lewis acid (see Japanese Patent Publication for Opposition Purpose (hereunder referred to as "J.P. KOKOKU") No. Sho 56-5386);
3. Those based on the condensation by heating of an aromatic compound with sulfuric acid or an aromatic sulfonic acid (see J.P. KOKOKU No. Sho 55-44743);
4. Those based on the condensation of an aromatic compound with an aromatic sulfonic acid in the presence of phosphorus pentoxide (see Japanese Patent Un-examined Publication (hereunder referred to as "J.P. KOKAI") No. Sho 60-92256);
5. Those based on the condensation by heating of an aromatic compound with sulfuric acid or an aromatic sulfonic acid in the presence of an ultra-strong acidic resin (see J.P. KOKAI Nos. Sho 57-85363); and
6. Those based on the condensation by heating of an aromatic compound with sulfuric acid or an aromatic sulfonic acid in the presence of a heteropoly-acid (see J.P. KOKAI Nos. Sho 63-23854 and Sho 63-68556).

Among these methods for preparing aromatic sulfones, most preferred are those in which the condensation reaction of an aromatic compound with sulfuric acid or an aromatic sulfonic acid is utilized because of the low cost of the principal starting materials, but these methods suffer from various problems. For instance, the reaction rate of the condensation by heating in the absence of any catalyst (Method 3) is low and thus the productivity thereof is insufficient; it is difficult to repeatedly use phosphorus pentoxide in the condensation reaction in the presence of phosphorus pentoxide (Method 4), which leads to an increase in cost; in the condensation reaction in the presence of an ultra-strong acidic resin (Method 5), the ultra-strong acidic resin is not only very expensive but also has short period of endurance, which also leads to an increase in cost; and in the condensation reaction in the presence of a heteropoly-acid (Method 6), the reaction rate decreases at the end of the reaction, the recovery of the expensive heteropoly-acid is low and the operation for the recovery can be performed only with great difficulties.

The present invention is concerned with a condensing method carried out in the presence of a heteropoly-acid. Therefore, problems associated with such techniques will be described in more detail below.

Upon condensation by heating of an aromatic compound with sulfuric acid or an aromatic sulfonic acid in the presence of a heteropoly-acid, it is observed that the catalytic activity of the heteropoly-acid is gradually lowered. When the reduction in the catalytic activity is analytically investigated, it is found that, under an extreme dehydration condition, the heteropoly-acid irreversibly causes separation into tungsten oxide or molybdenum oxide and phosphoric acid or silisic acid serving as a core compound (hetero compound). The heteropoly-acid from which the hetero compound is released is composed of tungstic acid or molybdic acid which would be further converted into more stable forms which may be regarded as tungsten oxide or molybdenum oxide if it is heated. The tungsten oxide or molybdenum oxide as the product of such an irreversible decomposition of the heteropoly-acid does not show catalytic activity at all. In addition, they are fine colloidal materials which are dissolved in neither the reaction system nor water and hence they makes it very difficult to perform separation and recovery of the heteropoly-acid and/or the resulting aromatic sulfone from the reaction mixture. Further, it is difficult to regenerate heteropoly-acids from these colloidal materials. The aforesaid problems associated with the irreversible decomposition or change of the heteropoly-acid can be summarised as follows:

(1) reduction in productivity due to the deterioration of the catalytic activity of the heteropoly-acid;
(2) reduction in workability of separation of the heteropoly-acid from the desired product and recovery of the acid and the desired products because of the formation of colloidal materials; and
(3) lowering of the recovery efficiency of the heteropoly-acid.

## SUMMARY OF THE INVENTION

The inventors have found that all of the foregoing drawbacks associated with the conventional

techniques for preparing aromatic sulfones can effectively be eliminated if a proper amount of orthophosphoric acid is put in contact with the reaction system when an aromatic compound is condensed, by heating, with sulfuric acid or an aromatic sulfonic acid in the presence of a heteropoly-acid, whereby the irreversible decomposition of the heteropoly-acid discussed above can be suppressed. Moreover, it has also been found that such an effect of controlling the decomposition is one specific to the orthophosphoric acid and cannot be attained by other condensed type phosphorus cmpounds such as pyrophosphoric acid, metaphosphoric acid or phosphorus pentoxide. It is considered that this effect can be achieved as a result of the inhibition of the escape of the hetero compound such as orthophosphoric acid or silisic acid derived from the heteropoly-acid through the shifting of the chemical equilibrium due to the excess of orthophosphoric acid the escape of the hetero compound being a first step of the irreversible decomposition of the heteropoly-acid.

According to the first aspect of the present invention, there is provided a method for preparing aromatic sulfones which comprises condensing, by heating, an aromatic compound with sulfuric acid or an aromatic sulfonic acid in the presence of a heteropoly-acid and orthophosphoric acid.

According to the second aspect of the present invention, there is provided a method for preparing aromatic sulfones which comprises the steps of: condensing, by heating, an aromatic compound with sulfuric acid or an aromatic sulfonic acid in the presence of a heteropoly-acid and orthophosphoric acid; and adding water and optionally an aromatic compound to the resulting reaction mixture to separate it into (i) a mixture of the aromatic sulfone and unreacted aromatic compound and (ii) an aqueous solution containing the heteropoly-acid, orthophosphoric acid and aromatic sulfonic acid and to thus recover the same.

According to the third aspect of the present invention, there is provided a method for preparing aromatic sulfones wherein, in the foregoing methods for preparing aromatic sulfones, the aqueous solution containing the heteropoly-acid, orthophosphoric acid and aromatic sulfonic acid which is separated and recovered according to the aforesaid method is employed as the heteropoly-acid and orthophosphoric acid.

## DESCRIPTION OF THE PREFERRED EMBODIMENTS

The aromatic compounds and aromatic sulfonic acids as used herein and the aromatic sulfones are, for instance, represented by the following general formulae (I) and (II) and (III) or (IV) respectively:

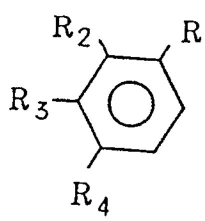
(I)

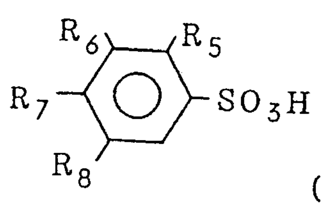
(II)

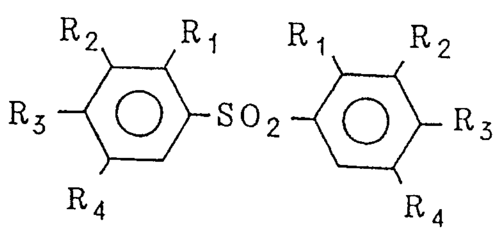
(III)

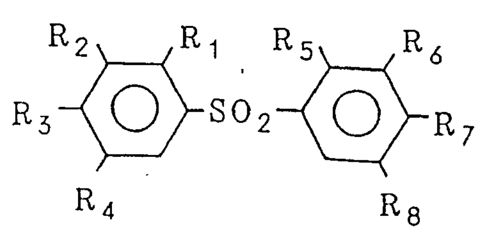
(IV)

(in the general formulae (I) to (IV), $R_1$ to $R_8$ each represents a hydrogen atom, a halogen atom, a hydroxyl group, an alkyl group, a phenyl group, a substituted phenyl group, an alkyloxy group or an aryloxy group).

Specific examples of the aromatic compounds represented by the general formula (I) are benzene, chlorobenzene, dichlorobenzene, trichlorobenzene, bromobenzene, dibromobenzene, phenol, toluene, xylene, ethylbenzene, isopropylbenzene, sec-butylbenzene, tertbutylbenzene, biphenyl, 4-chlorobiphenyl, 4-bromobiphenyl, 2-hydroxybiphenyl, 4-hydroxybiphenyl, 4-carboxybiphenyl, anisole, diphenyl ether, 4-chlorodiphenyl ether, 4-bromodiphenyl ether, 4-carboxydiphenyl ether, 4-nitrodiphenyl ether, o-cresol, 2,5-xylenol, 2,6-xylenol or 3,5-dimethyl-4-hydroxybiphenyl. Specific examples of the aromatic sulfonic acids represented by the general formula (II) and the aromatic sulfones represented by the general formula (III) or (IV) include all the corresponding derivatives derived from the foregoing compounds listed above as specific examples of Compound (I). Examples of industrially important aromatic sulfones represented by the general formula (III) or (IV) are diphenyl sulfone, 4,4'-dichlorodiphenyl sulfone, 2,4,5,4'-tetrachlorodiphenyl sulfone, 4,4'-dihydroxydiphenyl sulfone, 4,4'-dimethyldiphenyl sulfone, 3,4,3',4'-tetramethyldiphenyl sulfone, 4,4'-diphenyldiphenyl sulfone, 4,4'-di-(4-bromophenyl)-diphenyl sulfone, 3,3'-diphenyl-4,4'-dihydroxydiphenyl sulfone, 4,4'-di-(4-hydroxyphenyl)-diphenyl sulfone, 4,4'-di-(4-carboxyphenyl)-diphenyl sulfone, 4,4'-diphenoxydiphenyl 4,4'-diphenoxydiphenyl sulfone, 4,4'-di-(4-carboxyphenoxy)-diphenyl sulfone, 4,4'-di-(4-nitrophenoxy)-diphenyl sulfone, 3,3'-dimethyl-4,4'-dihydroxydiphenyl sulfone or 3,5,3',5'-tetramethyl-4,4'-dihydroxydiphenyl sulfone.

Specific examples of the heteropoly-acids are phospho-tungstic acid ($H_3PO_4$-12$WO_3$-x$H_2O$), silico-tungstic acid ($H_4SiO_4$-12$WO_3$-x$H_2O$) or phospho-molybdic acid ($H_3PO_4$-12$MoO_3$-x$H_2$ O). These heteropoly-acids are in general added to the reaction system in an amount ranging from 0.1 to 10% by weight. On the other hand, orthophosphoric acid is added to the reaction system in an amount ranging from 1 to 500% by weight on the basis of the weight of the heteropoly-acid. The heteropoly-acid and orthophosphoric acid are preferably added to the system in the form of an aqueous solution.

The condensation reaction is preferably carried out at a temperature ranging from about 120 to 230 °C while water is azeotropically removed. Most preferably, the aromatic compound and water used in the reaction are smoothly azeotropically distilled off under the temperature condition of the condensation reaction. However, the boiling points of the aromatic compounds employed in the reaction vary depending on the kinds thereof. Therefore, some consideration is needed in order to smoothly boil the reaction mixture at a proper temperature of the condensation reaction. If the boiling point of the aromatic compound is too low to achieve a satisfactory reaction temperature, the smooth azeotropic distillation of them can be achieved, for instance, by applying a pressure to the reaction system or by adjusting the rate of addition of the aromatic compound so as to maintain the reaction temperature in proportion to the progress of the reaction. On the other hand, the boiling point of the aromatic compound is too high to cause smooth boiling at a proper temperature of the reaction, the smooth azeotropic distillation thereof can be achieved, for instance, by reducing the reaction pressure or by adding another low boiling point inert solvent in an amount which makes it possible to maintain the reaction temperature at a preferred level. Examples of preferred such inert solvents are hexane, heptane, octane, decane, cyclohexane, methylcyclohexane, dichloroethane, chlorobenzene, dichlorobenzene, nitrobenzene or sulfolane.

The progress of the reaction can be monitored by determining the amount of water removed at any stage. The condensation reaction can be stopped at the time when the rate of the aromatic sulfone produced with respect to sulfuric acid or the aromatic sulfonic acid employed reaches 50 to 98%. It is possible to recover, from the resulting reaction mixture, the aromatic sulfone produced, and the unreacted aromatic sulfonic acids and aromatic compounds as well as the heteropoly-acid and orthophosphoric acid. One example of most preferred methods for recovering these compounds is to add water and optionally an additional amount of an aromatic compound. Whereby the aromatic compounds are dissolved in the aromatic sulfone to give an oil phase while the aromatic sulfonic acid, heteropoly-acid and orthophosphoric acid are dissolved in water to provide an aqueous phase. Then the oil phase is separated from the aqueous phase. The oil phase is further washed with water and then the aromatic sulfone can be separated from the aromatic compound by any known distillation or crystallization technique. On the other hand, the aqueous phase can be used in the subsequent condensation reaction as such or after the removal of water from the water phase or the concentration of the same by distillation. In this respect, the catalytic activity of this reaction system remains unchanged even if a heteropoly-acid and orthophosphoric acid are not additionally added thereto. It is found in terms of the recycle test of the aqueous phase, that the aqueous phase can repeatedly or continuously employed over 5 to 25 times. The solution recovered sometimes has a tinge of pale blue which is the color developed by the reduced heteropoly-acid. This solution can completely be regenerated by the addition of a proper amount of hydrogen peroxide.

The present invention will be explained in more detail with reference to the following specific Examples and Comparative Examples.

Example 1

To a 2,000 ml inner volume, four-necked flask of hard glass equipped with a stirrer, a thermometer, a dropping funnel and a rectifying column which has an inner diameter of 30 mm and which is packed with Raschig rings having a diameter of 6 mm up to a height of 250 mm thereof, there were added 600 g of

98% sulfuric acid and 843 g of monochlorobenzene. A separatory tube and a condenser capable of separating monochlorobenzene from water and hence removing water were disposed at the upper portion of the rectifying column and the contents of the flask was heated by an electric heating mantle of about 400 W with stirring. The contents started boiling as soon as the temperature thereof reached about 130 °C and water was azeotropically removed. When the amount of water removed reached about 100 ml, a mixed solution containing 60 g of phospho-tungstic acid, 30 g of orthophosphoric acid and 40 g of water was dropwise added to the contents through the dropping funnel. As the water was further azeotropically removed, the temperature of the contents rose. Thus, the temperature was adjusted by the dropwise addition of monochlorobenzene through the dropping funnel so that the temperature thereof was held at 190 °C. About 20 hours after the initiation of heating, the total amount of water removed reached about 240 ml. At this stage, 500 ml of water and 1,500 g of monochlorobenzene were added to a 5,000 inner volume flask of hard glass provided with a stirrer, a reflux condenser, a thermometer and an inlet for pouring at the upper portion and an outlet for withdrawing at the lower portion and the foregoing reaction mixture was poured into the flask through the inlet with vigorous stirring. The flask was heated to boil the contents thereof. 30 Minutes after the interruption of heating and stirring, the lower phase was withdrawn through the outlet, this phase being hereunder regarded as a Recovered Liquid 1. This was an aqueous solution containing the phosphotungstic acid, orthophosphoric acid and chlorobenzene sulfonic acid as well as a small amount of the resulting sulfone. 500 ml of water was further added to the flask and the contents was again heated with stirring. About 30 minutes thereafter, the heating and stirring were interrupted and the contents were again allowed to stand for 30 minutes to give, in this case, an oil phase as the lower phase and an aqueous phase as the upper phase. The oil phase was dehydrated, filtered and then cooled to give precipitates of 4,4-dichlorodiphenyl sulfone. The precipitates were purified in a known manner to thus give about 1,100 g of white crystals having a melting point of 149 °C. The upper aqueous phase was removed from the flask, this phase being hereunder regarded as Recovered Liquid 2.

Example 2

To the same 2,000 ml flask used in Example 1, there were added 450 g of 98% sulfuric acid and 700 g of monochlorobenzene and they were reacted according to the same procedures used in Example 1. When the amount of water azeotropically removed reached 75 ml, Recovered Liquid 1 obtained in Example 1 was dropwise added through a dropping funnel while water was removed successively. After the completion of the dropwise addition, the temperature of the contents rose gradually. As soon as the temperature reached 190 °C, it was adjusted by the dropwise addition of monochlorobenzene as in Example 1. About 27 hours after the initiation of heating, the rate of the progress of reaction reached the same level as that observed in Example 1 after 20 hours. At this stage, Recovered Liquid 2 obtained in Example 1 and 1,500 g of monochlorobenzene were added to the same 5,000 ml flask used in Example 1 and the same procedures used in Example 1 were repeated to thus give Recovered Liquids 1-2 and 2-2 and 1,100 g of the desired compound having a melting point of 149 °C.

Example 3

The same procedures used in Example 2 were repeated except that Recovered Liquid 1-2 and Recovered Liquid 2-2 were substituted for Recovered Liquids 1 and 2 respectively to thus give the same amount of the desired compound and Recovered Liquids 1-3 and 2-3.

Example 4

The procedures used in Examples 2 and 3 were repeated in order over 9 times. The same products as those obtained in Examples 1 to 3 were obtained.

Comparative Example 1

The same procedures used in Example 1 were repeated except that orthophosphoric acid was not employed. Thus, 1,030 g of the desired product was obtained. Subsequently, the same procedures used in Examples 2 and 3 were repeated and 1,020 g and 970 g of the desired products were obtained respectively. However, a substantial amount of colloidal materials of blueish white gathered at the oil/water interface during the separation operation, this making the operation difficult compared with those in Examples 1 to 4.

Example 5

To a 3,000 ml inner volume, four-necked flask of hard glass equipped with a stirrer, a thermometer, a dropping funnel and a rectifying column which has an inner diameter of 30 mm and which is packed with Raschig rings having a diameter of 6 mm up to a height of 250 mm thereof, there were added 1,800 g of o-phenylphenol and 300 g of monochlorobenzene. A separating tube and a condenser capable of separating water from
monochlorobenzene and removing them were disposed at the upper portion of the rectifying column. A means for establishing vacuum was connected to the upper portion of the condenser. The flask was heated and stirring was initiated when the contents were molten at about 60 °C. At this temperature, 400 g of 98% sulfuric acid was dropwise added to the contents through the dropping funnel over about 30 minutes. After the completion of the dropwise addition, the temperature of the contents was raised up to 100 °C and after one hour, a mixed solution comprising 48 g of phospho-tungstic acid, 24 g of orthophosphoric acid and 30 g of water was dropwise added thereto through the dropping funnel. At this stage, the means for establishing vacuum connected to the condenser at its upper portion was started so that a vacuum in the order of 200 torr was steadily established in the flask. The flask was heated to cause boiling of the contents. The water and monochlorobenzene were removed so that the contents of the flask boiled at 140 °C. The amount of water removed exceeded 165 g, 20 hours after the contents being controlled to boil at 140 °C. After the interruption of the means for establishing vacuum, 600 g of monochlorobenzene and 400 g of water were slowly dropwise added to the flask through the dropping funnel. The heating operation was controlled so that the contents gently boiled during the dropwise addition. After the dropwise addition, the contents was stirred for additional one hour and then the flask was slowly cooled. The contents of the flask was composed of precipitates of the desired product, i.e., 3,3'-diphenyl-4,4'-dihydroxydiphenylsulfone, an oil phase and aqueous phase. The oil phase comprised monochlorobenzene in which o-phenylphenol and isomers of the sulfone were dissolved while the aqueous phase comprised water in which the phospho-tungstic acid, orthophosphoric acid and o-phenylphenol sulfonic acid are dissolved. This mixture consisting of the precipitates, the oil phase and the aqueous phase was filtered by suction and the filtrate was regarded as Recovered Liquid 3. The resulting filter cake was washed with 600 g of monochlorobenzene and 400 g of water and the wash liquid was regarded as Recovered Liquid 4. The filter cake was purified in the usual manner to thus give about 950 g of the desired product having a metling point of 250 °C.

Example 6

To the same flask used in Example 5, there were added Recovered Liquid 3 and 816 g of o-phenylphenol. The flask was heated with stirring to azeotropically remove water. When about 380 g of water was removed, 240 g of 98% sulfuric acid was dropwise added thereto through the dropping funnel and the means for establishing vacuum was started so that the pressure of 200 torr was established in the flask. Thereafter the reaction was carried out as in Example 5. Then Recovered Liquid 5 was dropwise added through the dropping funnel in place of the addition of 600 g of monochlorobenzene and 400 g of water in Example 5. Thereafter, the same procedures used in Example 5 were repeated and thus about 920 g of the desired product having a melting point of 250 °C was obtained and Recovered Liquid 3-6 and 4-6 were also obtained instead of the Recovered Liquids 3 and 4.

Example 7

The same procedures used in Example 6 were repeated except that Recovered Liquids 3-6 and 4-6 were substituted for Recovered Liquids 3 and 4 respectively. Thus, Recovered Liquids 3-7 and 4-7 and about 925 g of the desired product having a melting point of 250 °C were obtained. As Recovered Liquid 3-7 had a tinge of slight blue, it was regenerated by the addition of about 1.2 ml of a 30% aqueous solution of hydrogen peroxide.

Example 8

The same procedures used in Examples 6 and 7 were repeated except that Recovered Liquids 3-7 and 4-7 were used and thus Recovered Liquids 3-8 and 4-8 as well as about 920 g of the desired product having a melting point of 250 °C were obtained.

Comparative Example 2

The procedures used in Example 5 were repeated except that orthophosphoric acid was not employed and thus 910 g of the desired product and Recovered Liquids 3-9 and 4-9 were obtained. It should be noted that the filter paper was clogged with colloidal materials formed, during filtering by suction, this making it difficult to some extent to perform the filtering operation.

Comparative Example 3

The procedures used in Example 6 were repeated except that Recovered Liquids 3-9 and 4-9 obtained in Comparative Example 2 were employed and thus 880 g of the desired product as well as Recovered Liquids 3-10 and 4-10 were obtained. The filter paper was severely clogged with colloidal materials formed, this making the filtering operation very difficult.

Example 9

To the same flask used in Example 1 there were added 848 g of o-xylene and 500 g of 98% sulfuric acid and the flask was heated with stirring to slowly raise the temperature of the contents. When the contents started boiling, water was removed as an azeotropic mixture with o-xylene. When the amount of water removed reached about 90 g, a mixed solution comprising 50 g of phospho-tungstic acid, 15 g of orthophosphoric acid and 30 g of water was dropwise added to the contents through the dropping funnel. As water was removed successively, the temperature of the contents rose and reached 160 °C. Thus, the temperature was adjusted by the dropwise addition of o-xylene from the dropping funnel so that it did not exceed 160 °C. About 20 minutes after the temperature of the contents reaching 160 °C, the amount of water removed exceeded 200 g. At this stage, 1,000 g of o-xylene and 500 g of water were added to the same flask having an inner volume of 5,000 ml capable of separating liquids as that used in Example 1 and the foregoing reaction mixture was poured into the flask through the inlet with vigorous stirring. This flask was heated to cause boiling of the contents. The heating and the stirring were interrupted and 30 minutes thereafter the lower phase was withdrawn, which was regarded as Recovered Liquid 6. 500 g of water was again added to the flask and the same operations were repeated to give Recovered Liquid 7. The resulting oil phase was dehydrated and cooled to give precipitates of the intended compound, 3,3',4,4'-tetramethyl diphenyl sulfone. The precipitates were purified in the usual manner to obtain about 910 g of the desired product having a melting point of 167 °C.

Example 10

742 g of o-xylene and 400 g of 98% sulfuric acid were added to the same flask used in Example 9 and the same procedures used in Example 9 were repeated. When the amount of water removed reached 70 g, Recovered Liquid 5 obtained in Example 9 was dropwise added to the contents of the flask through the dropping funnel while water was azeotropically removed successively. The same procedures used in Example 9 were again repeated to complete the reaction. At this stage, 1,000 g of o-xylene and Recovered Liquid 6 obtained in Example 9 were added to a 5,000 ml volume flask capable of separating liquids and the same operations used in Example 9 were repeated to give Recovered Liquid 5-10. Likewise, Recovered Liquid 7 was added to the flask to obtain Recovered Liquid 6-10. Then 500 ml of water was added to give Recovered Liquid 7-10. In this Example, 905 g of the desired product having a melting point of 167 °C was recovered.

Example 11

The same procedures used in Example 10 were repeated except that Recovered Liquids 5-10, 6-10 and 7-10 were substituted for Recovered Liquids 5, 6 and 7 respectively and thus Recovered Liquids 5-11, 6-11 and 7-11 as well as 915 g of the desired product having a melting point of 167 °C were recovered. In this respect, Recovered Liquid 5-11 had a tinge of blue and thus it was regenerated by the addition of 0.8 ml of a 30% aqueous solution of hydrogen peroxide. Thus Recovered Liquid 5-11 could withstand the subsequent repeated use in the condensation reaction.

Comparative Example 4

The same procedures used in Example 9 were repeated except that orthophosphoric acid did not employed and thus Recovered Liquids 5-12, 6-12 and 7-12 as well as 890 g of the desired product were

obtained.

Comparative Example 5

The same procedures used in Example 10 were repeated except that Recovered Liquids 5-12, 6-12 and 7-12 were substituted for Recovered Liquids 5, 6 and 7 respectively and thus respective Recovered Liquids and 885 g of the desired product were obtained. However, it should be noted that colloidal materials of pale blue gathered at the water/oil interface during the separation of liquids, which made the separating operation very difficult.

## Claims

1. A method for preparing an aromatic sulfone comprising the step of condensing, by heating, an aromatic compound with sulfuric acid or an aromatic sulfonic acid in the presence of a heteropoly-acid and orthophosphoric acid.

2. The method of claim 1 wherein the heteropoly-acid is added to the reaction system in an amount ranging from 0.1 to 10% by weight and the orthophosphoric acid is added in an amount ranging from 1 to 500% by weight on the basis of the total weight of the heteropoly-acid.

3. The method of claim 1 wherein the heteropoly-acid and orthophosphoric acid are added to the reaction system in the form of an aqueous solution.

4. The method of claim 1 wherein the heteropoly-acid is phospho-tungstic acid.

5. The method of claim 1 wherein the condensation reaction by heating is carried out at a temperature ranging from 120 to 230 °C while water is azeotropically removed.

6. A method for preparing an aromatic sulfone comprising the steps of: condensing, by heating, an aromatic compound with sulfuric acid or an aromatic sulfonic acid in the presence of a heteropoly-acid and orthophosphoric acid adding water to the resulting reaction mixture to separate it into (i) a mixture of the aromatic sulfone and unreacted aromatic compound and (ii) an aqueous solution containing the heteropoly-acid, orthophosphoric acid and aromatic sulfonic acid, to thus recover the same.

7. The method of claim 6 wherein the heteropoly-acid is added to the reaction system in an amount ranging from 0.1 to 10% by weight and the orthophosphoric acid is added in an amount ranging from 1 to 500% by weight on the basis of the total weight of the heteropoly-acid.

8. The method of claim 6 wherein the aqueous solution containing the heteropoly-acid, orthophosphoric acid and aromatic sulfonic acid which is separated and recovered according to the method as set forth in claim 6 is employed as the heteropoly-acid and orthophosphoric acid.

9. The method of claim 6 wherein the heteropoly-acid is phospho-tungstic acid.

10. The method of claim 6 wherein the condensation reaction by heating is carried out at a temperature ranging from 120 to 230 °C while water is azeotropically removed.

11. A method for preparing an aromatic sulfone comprising the steps of: condensing, by heating, an aromatic compound with sulfuric acid or an aromatic sulfonic acid in the presence of a heteropoly-acid and orthophosphoric acid; adding water and an aromatic compound to the resulting reaction mixture to separate it into (i) a mixture of the aromatic sulfone and unreacted aromatic compound and (ii) an aqueous solution containing the heteropoly-acid, orthophosphoric acid and aromatic sulfonic acid, to thus recover the same.

12. The method of claim 11 wherein the heteropoly-acid is added to the reaction system in an amount ranging from 0.1 to 10% by weight and the orthophosphoric acid is added in an amount ranging from 1 to 500% by weight on the basis of the total weight of the heteropoly-acid.

**13.** The method of claim 11 wherein the aqueous solution containing the heteropoly-acid, orthophosphoric acid and aromatic sulfonic acid which is separated and recovered according to the method as set forth in claim 11 is employed as the heteropoly-acid and orthophosphoric acid.

**14.** The method of claim 11 wherein the heteropoly-acid is phospho-tungstic acid.

**15.** The method of claim 11 wherein the condensation reaction by heating is carried out at a temperature ranging from 120 to 230 °C while water is azeotropically removed.

European Patent Office

# EUROPEAN SEARCH REPORT

Application Number

**EP 90 10 5756**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | DE-A-3 723 401 (NEW JAPAN CHEMICAL CO.)<br>* Claims<br>* & JP-A-63 23 854, JP-A-63 68 556 (Cat. D)<br>- - - - - | 1,4,6,9, 11,14 | C 07 C 315/00 |

| TECHNICAL FIELDS SEARCHED (Int. Cl.5) |
|---|
| C 07 C 315/00<br>C 07 C 317/00 |

The present search report has been drawn up for all claims

| Place of search | Date of completion of search | Examiner |
|---|---|---|
| The Hague | 13 November 90 | VAN GEYT J.J.A. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document